# EUROPEAN PATENT SPECIFICATION

(11) **EP 3 111 929 B1**
(45) Date of publication and mention of the grant of the patent: **29.08.2018**
(21) Application number: 15811973.5
(22) Date of filing: 19.06.2015
(51) Int. Cl.: A61K 9/70, A61K 31/4985, A61K 47/32, A61K 47/36

(54) **ORAL DISINTEGRATING FILM FORMULATION CONTAINING TADALAFIL AND PREPARATION METHOD THEREFOR**
IM MUND ZERFALLENDE FILMFORMULIERUNG MIT TADALAFIL UND HERSTELLUNGSVERFAHREN DAFÜR
COMPOSITION PHARMACEUTIQUE PELLICULÉE À DÉSINTÉGRATION ORALE CONTENANT DU TADALAFIL ET SA MÉTHODE DE PRÉPARATION

(30) Priority: 24.06.2014 KR 20140077569
(43) Date of publication of application: 04.01.2017
(73) Proprietor: Wooshin Labottach Co., Ltd., Seoul (KR)
(72) Inventor: NAM, Tack-Soo, Seoul 150-888 (KR)
(74) Representative: Paulraj, Leonita Theresa
(86) International application number: PCT/KR2015/006223
(87) International publication number: WO 2015/199380

(56) References cited:
- WO-A1-2013/085276
- WO-A2-01/08688
- WO-A2-2012/053006
- KR-A- 20130 003 511
- KR-B1- 101 407 922

## Description

### TECHNICAL FIELD

The present invention relates to an orally disintegrating film formulation comprising tadalafil as an active ingredient and a process for preparing the same. More specifically, the present invention relates to a tadalafil-containing orally disintegrating film formulation comprising a combination of pullulan and polyvinylpyrrolidone as a film forming agent and a process for preparing the same.

### BACKGROUND ART

Erectile dysfunction (ED) is a common disease that occurs to about 10% of adult males, and it has been reported that about 150 million worldwide, 30 million in the US and 2.5-3 million in Korea suffer therefrom. Due to the increase of aging population caused by aging phenomenon of society and the stress and overwork of the industrialization and radical changes of society, the number of the patients suffering from erectile disorder tends to increase sharply. In 2020, the number of patients will be expected to be more than 300 million that is more than double what it is.

Tadalafil has the following structure, and its chemical name is (6R, 12aR)-6-(1,3-benzodioxol-5-yl)-2,3,6,7,12,12a-hexahydro-2-methyl-pyrazino[1',2':1,6]p yrido[3,4-b]indole-1,4-dion. Tadalafil acts as an inhibitor of cyclic guanosine 3',5'-monophosphate phosphodiesterase type 5 (cGMP PDE5), which is similar to sildenafil (Viagra) and vardenafil (Levitra), but as its selectivity of PDE5/PDE6 is better than sildenafil, recently it has been noticed as a substance for treating ED.

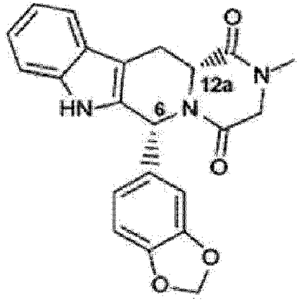

Meanwhile, recently considering the situation, portability, etc. that ED drugs are used, the researches for applying to film formulation instead of the existing oral formulations such as pills, capsules, etc. have been made. As to film formulation, even the patients who have difficulty in swallowing or eating drugs can take drugs easily, and it also has a merit that drugs can be orally administered without water. Generally, in case of film formulations, active ingredients are produced by dispersion or suspension without dissolving the active ingredients, so it can be more effective to produce sparingly water soluble substances such as tadalafil by film formulation.

However, tadalafil is a drug having very low solubility, which belongs to BCS (Biopharmaceutics Classification System) Class II or IV. It has been reported that it is actually water-insoluble and only a bit amount is dissolved in several organic solvent such as methanol, ethanol and acetone. Especially, there is a problem that the dissolution rate of tadalafil is decreased due to the binding between polymer matrix and tadalafil, in the polymer base of a tadalafil-containing orally disintegrating film formulation. Accordingly, in preparing a tadalafil-containing orally disintegrating film formulation, it is an overriding concern to increase the solubility of a drug (active ingredient) for raising the dissolution rate thereof. In this regard, one of the ways to improve the solubility of a sparingly-soluble active material is to increase the particle surface area of the active material by grinding or micronizing the material (WO 01/08688). More specifically, the particle size of tadalafil should be decreased below 40 µm to reach the aimed solubility or release, and the tadalafil having a particle size of 40 µm or less is used for the commercially available tablet (Cialis Tablet).

Meanwhile, it is desirable that a tadalafil-containing orally disintegrating film formulation is bioequivalent to the commercially available tablet (i.e., Cialis Tablet). And also, it is required that there is no substantial buccal absorption which may be brought about according to the characteristics (i.e., rapid disintegration) of an orally disintegrating film formulation. However, according to polymer bases of the orally disintegrating film formulation, the resulting formulations not only exhibit quite different dissolution patterns but also cause low-stability problems.

### DISCLOSURE

### Technical Problem

The present inventor carried out various researches in order to develop an orally disintegrating film formulation which exhibits bioequivalence to a tadalafil-containing tablet, while showing no buccal absorption. Especially, the present inventor evaluated the pharmacokinetic characteristics, stabilities, etc. of tadalafil, using various polymer bases (i.e., film forming agents). Surprisingly, the present inventor found that, when a combination of the specific film forming agents, i.e., a combination of pullulan and polyvinylpyrrolidone, is used as a film forming agent, the resulting orally disintegrating film formulation exhibits not only bioequivalence to a tadalafil-containing tablet, while showing no buccal absorption, but also excellent stability.

Therefore, it is an object of the present invention to provide a tadalafil-containing orally disintegrating film formulation comprising a combination of pullulan and polyvinylpyrrolidone as a film forming agent.

And also, it is another object of the present invention to provide a process for preparing the tadalafil-containing orally disintegrating film formulation..

### Technical Solution

In accordance with an aspect of the present invention, there is provided an orally disintegrating film formulation, comprising tadalafil as an active ingredient and a combination of pullulan and polyvinylpyrrolidone as a film forming agent.

In the orally disintegrating film formulation of the present invention, said tadalafil may have d₉₀ particle size of 40 µm or less, preferably d₉₀ particle size ranging from 5 to 25 µm. And also, said pullulan may have weight average molecular weight ranging from 50,000 to 200,000, and said polyvinylpyrrolidone may have weight average molecular weight ranging from 700,000 to 2,500,000. In an embodiment, the weight ratio of tadalafil, pullulan and polyvinylpyrrolidone may be 1 : 1.8∼2.5 : 0.8∼1.3, preferably 1 : 2 : 1.

The orally disintegrating film formulation of the present invention may further comprise hydroxypropyl cellulose as a solubilizing agent. Said hydroxypropyl cellulose may be present in 1.5∼3 %(w/w) based on the total weight of the orally disintegrating film formulation.

The orally disintegrating film formulation of the present invention may also comprise one or more pharmaceutically acceptable excipients selected from the group consisting of a surfactant, a plasticizer, a sweetening agent, a flavoring agent, and a coloring agent. In an embodiment, the surfactant may be sodium lauryl sulfate; the plasticizer may be polyethylene glycol; and the sweetening agent may be a mixture of sucralose and mannitol.

In an embodiment of the present invention, there is provided an orally disintegrating film formulation, comprising tadalafil 15∼20 %(w/w), pullulan 30∼40 %(w/w), polyvinylpyrrolidone 15∼20 %(w/w), hydroxypropyl cellulose 1.5∼3 %(w/w), a surfactant 0.3∼1.0 %(w/w), a plasticizer 3∼7 %(w/w), a sweetening agent 18∼23 %(w/w), a flavoring agent 1.5∼3 %(w/w), and a coloring agent 1.5∼3 %(w/w), per unit formulation. In another embodiment of the present invention, there is provided an orally disintegrating film formulation, comprising tadalafil 20 mg, pullulan 40 mg, polyvinylpyrrolidone 20 mg, hydroxypropyl cellulose 2 mg, a surfactant 0.5 mg, a plasticizer 5 mg, a sweetening agent 22 mg, a flavoring agent 2 mg, and a coloring agent 2 mg, per unit formulation.

In accordance with another aspect of the present invention, there is provided a process for preparing an orally disintegrating film formulation, which comprises:
(a) dissolving or dispersing tadalafil, pullulan, polyvinylpyrrolidone, optionally a solubilizing agent, and a pharmaceutically acceptable excipient in ethanol or a mixture of ethanol and water to form a solution or a dispersion; and
(b) casting the solution or the dispersion obtained in the step (a) on a hydrophobic film, followed by drying the resultant.

In the process of the present invention, the weight ratio of ethanol and water in the mixture of ethanol and water may be 1 : 2.5∼5.0. And also, the step (b) may be performed by degassing the solution or the dispersion obtained in the step (a) to remove an air bubble, casting the resulting solution or dispersion on a hydrophobic film, and then drying the resultant.

### ADVANTAGEOUS EFFECTS

The orally disintegrating film formulation of the present invention may be formulated so as to exhibit bioequivalence to a tadalafil-containing tablet, while showing no buccal absorption. And also, the orally disintegrating film formulation of the present invention exhibits excellent stability.

### DESCRIPTION OF DRAWINGS

FIG. 1 shows the results of the comparative dissolution tests of the orally disintegrating film formulations prepared in Examples and the comparative formulation
FIG. 2 shows the plasma concentration profiles according to administering the orally disintegrating film formulation of the present invention and the comparative formulation.
FIG. 3 shows the plasma concentration profiles according to administering the orally disintegrating film formulation comprising only pullulan as a film forming agent; and the comparative formulation.

### BEST MODE

The present invention provides an orally disintegrating film formulation, comprising tadalafil as an active ingredient and a combination of pullulan and polyvinylpyrrolidone as a film forming agent.

In the orally disintegrating film formulation of the present invention, the tadalafil used as an active ingredient may be used in a therapeutically effective amount, for example in an amount ranging from 1 to 40 mg, preferably about 20 mg, per unit orally disintegrating film formulation, but not limited thereto. Said tadalafil may have d₉₀ particle size of 40 µm or less, preferably d₉₀ particle size ranging from 5 to 25 µm.

The orally disintegrating film formulation of the present invention comprises a combination of pullulan and polyvinylpyrrolidone as a film forming agent. It has been found by the present invention that, when the combination of pullulan and polyvinylpyrrolidone is used as a film forming agent, the resulting orally disintegrating film formulation exhibits not only bioequivalence to a tadalafil-containing tablet, while showing no buccal absorption, but also excellent stability. Said pullulan may have weight average molecular weight ranging from 50,000 to 200,000, preferably from 80,000 to 150,000. And also, said polyvinylpyrrolidone may have weight average molecular weight ranging from 700,000 to 2,500,000, preferably from 800,000 to 2,000,000. In order to formulate so as to exhibit bioequivalence to a tadalafil-containing tablet, the weight ratio of tadalafil, pullulan and polyvinylpyrrolidone is preferably 1 : 1.8∼2.5 : 0.8∼1.3, more preferably 1 : 2 : 1.

The orally disintegrating film formulation of the present invention may further comprise hydroxypropyl cellulose as a solubilizing agent. Said hydroxypropyl cellulose optimizes the interaction between tadalafil and the film forming agent; and increases the solubility of tadalafil in the solution containing the film forming agent during the preparation, thereby increasing the homogeneity thereof. Said hydroxypropyl cellulose may be present in 1.5∼3 %(w/w) based on the total weight of the orally disintegrating film formulation.

If necessary, the orally disintegrating film formulation of the present invention may further comprise a pharmaceutically acceptable excipient. For example, the pharmaceutically acceptable excipient may be one or more selected from the group consisting of a surfactant, a plasticizer, a sweetening agent, a flavoring agent, and a coloring agent.

Examples of the surfactant include sodium lauryl sulfate, sucrose esters (e.g., Dub SE 11S, Dub SE XS, etc.), preferably sodium lauryl sulfate. The surfactant may be used e.g., in an amount ranging from 0.3 to 1.0 %(w/w), preferably from 0.4 to 0.6 %(w/w), based on the total weight of the formulation, but not limited thereto.

Examples of the plasticizer include polyethylene glycol, sorbitol, maltitol, xylitol, glycerin, propylene glycol, starch syrup, glycerin, triacetin, glycerol oleate, sucrose fatty acid ester, medium chain fatty acids etc., preferably polyethylene glycol (for example, polyethylene glycol 200). The plasticizer may be used e.g., in an amount ranging from 3 to 7 %(w/w), preferably from 3 to 5 %(w/w), based on the total weight of the formulation, but not limited thereto.

Examples of the sweetening agent include sucralose, mannitol, maltose, fructose, galactooligosaccharide, galactose, fructooligosaccharide, dextrin, neohesperidin, lactose, glucose, sorbitol, xylitol, inositol, aspartame, stevioside, acesulfame potassium, saccharin sodium, lemon oil, etc., preferably a combination of sucralose and mannitol. The sweetening agent may be used e.g., in an amount ranging from 18 to 23 %(w/w), preferably from 19 to 21 %(w/w), based on the total weight of the formulation, but not limited thereto.

Examples of the flavoring agent include any components conventionally used in the oral pharmaceutical formulation, such as fruit flavor, peppermint flavor, orange flavor, etc., without limitation. And also, examples of the coloring agent include any components conventionally used in the oral pharmaceutical formulation, such as natural pigment, tar pigment, blue No. 1, etc., without limitation. The flavoring agent and the coloring agent may be used in appropriate amounts, which can be appropriately determined by a skilled person in the art.

In an embodiment of the present invention, there is provided an orally disintegrating film formulation, comprising tadalafil 15∼20 %(w/w), pullulan 30∼40 %(w/w), polyvinylpyrrolidone 15∼20 %(w/w), hydroxypropyl cellulose 1.5∼3 %(w/w), a surfactant 0.3∼1.0 %(w/w), a plasticizer 3∼7 %(w/w), a sweetening agent 18∼23 %(w/w), a flavoring agent 1.5∼3 %(w/w), and a coloring agent 1.5∼3 %(w/w), per unit formulation. In another embodiment of the present invention, there is provided an orally disintegrating film formulation, comprising tadalafil 20 mg, pullulan 40 mg, polyvinylpyrrolidone 20 mg, hydroxypropyl cellulose 2 mg, a surfactant 0.5 mg, a plasticizer 5 mg, a sweetening agent 22 mg, a flavoring agent 2 mg, and a coloring agent 2 mg, per unit formulation.

The present invention also provides a process for preparing said orally disintegrating film formulation. That is, the present invention provides a process for preparing an orally disintegrating film formulation, which comprises: (a) dissolving or dispersing tadalafil, pullulan, polyvinylpyrrolidone, optionally a solubilizing agent, and a pharmaceutically acceptable excipient in ethanol or a mixture of ethanol and water to form a solution or a dispersion; and (b) casting the solution or the dispersion obtained in the step (a) on a hydrophobic film, followed by drying the resultant.

In the step (a), said tadalafil may have d₉₀ particle size of 40 µm or less, preferably d₉₀ particle size ranging from 5 to 25 µm. Said pullulan may have weight average molecular weight ranging from 50,000 to 200,000, preferably from 80,000 to 150,000. Said polyvinylpyrrolidone may have weight average molecular weight ranging from 700,000 to 2,500,000, preferably from 800,000 to 2,000,000. And also, the weight ratio of tadalafil, pullulan and polyvinylpyrrolidone is preferably 1 : 1.8∼2.5 : 0.8∼1.3, more preferably 1 : 2 : 1. The solubilizing agent may be hydroxypropyl cellulose, which may be used in 1.5∼3 %(w/w) based on the total weight of the orally disintegrating film formulation. The pharmaceutically acceptable excipient may be one or more selected from the group consisting of a surfactant, a plasticizer, a sweetening agent, a flavoring agent, and a coloring agent. The examples and amounts thereof are the same in the above. The weight ratio of ethanol and water in the mixture of ethanol and water used in the step (a) may be 1 : 2.5∼5.0, preferably about 1 : 3.

The step (b) may further comprise degassing the solution or the dispersion obtained in the step (a) to remove air bubbles. That is, the step (b) may be performed by degassing the solution or the dispersion obtained in the step (a) to remove an air bubble, casting the resulting solution or dispersion on a hydrophobic film, and then drying the resultant. The degassing may be carried out for example under vacuum. The hydrophobic film includes a plastic film conventionally used in the field of orally disintegrating film formulation, for example siliconized polyethylene terephthalate film (siliconized PET film). The drying may be carried out according to conventional methods, for example at a temperature ranging from 50 to 100 °C.

The orally disintegrating film formulation obtained according to the present invention may be prepared to have a thickness ranging from 100 to 120 *µ*m and the thickness may be controlled e.g., by adjusting the amount of the film forming agent in the dispersion. And also, the orally disintegrating film formulation obtained according to the present invention may be packaged using, for example, aluminum foil.

The present invention will be described in further detail with reference to the following examples and experimental examples. These examples and experimental examples are for illustrative purposes only and are not intended to limit the scope of the present invention.

### Example: Preparation of orally disintegrating film formulations

Orally disintegrating film formulations containing tadalafil were prepared according to the components and amounts shown in Table 1. The amounts of Table 1 represent the content (mg) in the unit formulation cut to a size of 7.5 cm².

The respective components of Table 1 were homogeneously mixed in a mixture of ethanol and water (3:1, weight ratio; in about 16 times the amounts of tadalafil). The tadalafil raw material having d₉₀ particle size of about 15 µm was used in the preparation. The respective resulting dispersions were stirred with a paddle under vacuum for about 5 hours to remove air bubbles. The degassed dispersions were casted on the sliconized polyethylene terephthalate film (SKC polyester film Inc.) to have a constant thickness, and then subject to hot-air drying. The resulting formulations were cut to a size of 7.5 cm² to prepare the orally disintegrating film formulations (thickness: about 120 *µ*m).

**<Table 1>**

| Component | Amount (7.5 cm², mg) | | | |
|---|---|---|---|---|
| | 1-1 | 1-2 | 1-3 | 1-4 |
| Tadalafil | 20.00 | 20.00 | 20.00 | 20.00 |
| Pullulan | 40.00 | - | - | 60.00 |
| Polyvinylpyrrolidone (PVP k-90) | 20.00 | 40.00 | 60.00 | - |
| Hydroxypropyl methylcellulose | - | 20.00 | - | - |
| Hydroxypropyl cellulose (Klucel-Exf) | 2.00 | 2.00 | 2.00 | 2.00 |
| Sodium lauryl sulfate | 0.50 | 0.50 | 0.50 | 0.50 |
| Polyethylene glycol (PEG 200) | 5.00 | 5.00 | 5.00 | 5.00 |
| Sucralose | 2.00 | 2.00 | 2.00 | 2.00 |
| Mannitol | 20.00 | 20.00 | 20.00 | 20.00 |
| Orange flavor | 2.00 | 2.00 | 2.00 | 2.00 |
| Blue No. 1 (0.1% solution) | 2.00 | 2.00 | 2.00 | 2.00 |
| Total | 113.50 | 113.50 | 113.50 | 113.50 |

### Experimental Example 1: Buccal absorption test

The orally disintegrating film formulation prepared in Example 1-1 was dissolved in the oral cavity for 30 seconds, and then spitted out. Blood samples were taken at 0, 2, 5, 10, 20, 40 minutes and 1 hour and then analyzed with a LC-MS-MS method to carry out the buccal absorption test. The results are shown in the following Table 2. The amounts of Table 2 represent the respective blood concentrations of tadalafil.

**<Table 2>**

| Time (minute) | Amount (ng/ml) |
|---|---|
| 0 | 0.00 |
| 2 | 0.00 |
| 5 | 0.00 |
| 10 | 0.00 |
| 20 | 0.00 |
| 40 | 0.00 |
| 60 | 0.00 |

As shown in the Table 2, no buccal absorption was observed in the orally disintegrating film formulation of the present invention.

### Experimental Example 2: Dissolution test

The dissolution tests of the respective orally disintegrating film formulations (n=6) prepared in Examples 1-1 to 1-3 were carried out using sinkers, according to the US Pharmacopeia. As a comparative formulation, the Cialis 20mg Tablet was used. 1000 ml of a phosphate buffer containing 0.5% sodium lauryl sulfate was used as a dissolution medium and the dissolution tests were performed at 37°C for 45 minutes while each paddle was rotated at 50 rpm. The respective amounts of tadalafil were measured with high performance liquid chromatography (HPLC) under the following conditions.
- Column: EnduroSil, C18, 250mm × 4.6 mm, 5µm, 120 A°
- Column temperature: 40°C
- Flow rate: 1.0 ml/min
- Injection volume: 20 µL
- Mobile phase: Acetonitrile and Solution A (45:55, v/v)

Solution A was prepared by mixing purified water and trifluoroacetic acid (1.0 ml) and then adding purified water thereto to give 1000 ml of the final volume.
- Detector: UV detector
- Wavelength: 285 nm

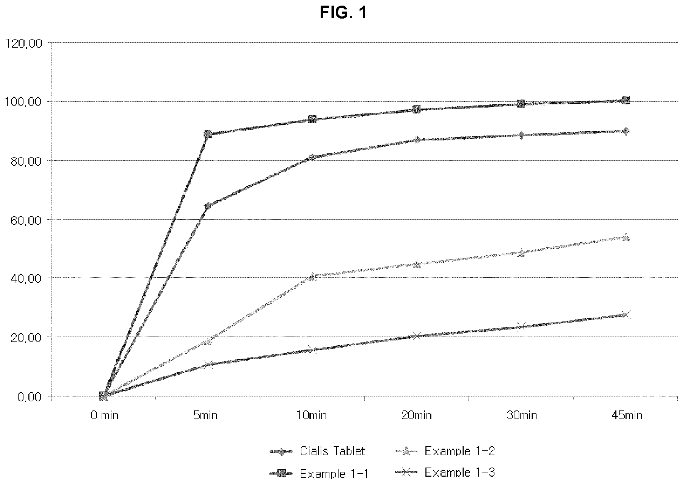

The results of said dissolution test were shown in the FIG. 1. The US Food and Drug Administration recommends that 85% or more of tadalafil should be dissolved out in 45 minutes. The results of FIG. 1 show that both the Example 1-1 formulation and the comparative formulation meet the requirement. Although the comparative formulation showed relatively lower dissolution rate than the Example 1-1 formulation, it is assumed due to shelf life elapse. In contrast, the Example 1-2 formulation which prepared by using the combination of polyvinylpyrrolidone and hydroxypropyl methylcellulose as a film forming agent; and the Example 1-3 formulation which prepared by using only polyvinylpyrrolidone as a film forming agent showed remarkably low dissolution rates.

### Experimental Example 3: Stability test

Stability test was carried out, while maintaining the orally disintegrating film formulation prepared in Example 1-1 under the conditions of 40°C and 75% relative humidity for 6 months. The stability was evaluated by measuring degradation product, total degradation products, disintegration time, and dissolution rate in 45 minutes. The degradation product and total degradation products were measured according to EP 7.4 under the following conditions.
- Column: EnduroSil, C18, 250mm × 4.6 mm, 5µm, 120 A°
- Column temperature: 40°C
- Flow rate: 1.0 ml/min
- Injection volume: 20 µL
- Mobile phase:
Mobile phase A was prepared by mixing purified water and trifluoroacetic acid (1.0 ml) and then adding purified water thereto to give 1000 ml of the final volume.
Mobile phase B: Acetonitrile

| Time (minute) | Mobile phase A | Mobile phase B |
|---|---|---|
| 0 - 3 | 85 | 15 |
| 3 - 30 | 85 -> 5 | 15 ->95 |
| 30 - 33 | 5 | 95 |
| 33 - 35 | 85 | 15 |
| 35 - 40 | 85 | 15 |

- Detector: UV detector
- Wavelength: 285 nm

The disintegration time was measured in purified water (900 ml) at 37°C, without using a disc, using standard assemblies according to EP 7.4. The dissolution rate in 45 minutes was measured according to the same methods of Experimental Example 2. The results are shown in the following Table 3.

**<Table 3>**

| | 0 month | 3 months | 6 months |
|---|---|---|---|
| Degradation product (NMT 0.1%) | <0.05 % | <0.05 % | <0.05 % |
| Total degradation product (NMT 0.5%) | <0.05 % | <0.05 % | <0.05 % |
| Disintegration time | 20 seconds | 19 seconds | 22 seconds |
| dissolution rate in 45 minutes | 100.7% | 98.5% | 97.0% |

As shown in the Table 3, the orally disintegrating film formulation of the present invention showed no substantial change when it maintained under the accelerated condition (40°C, 75% relative humidity) for 6 months.

### Experimental Example 4: Bioequivalence test

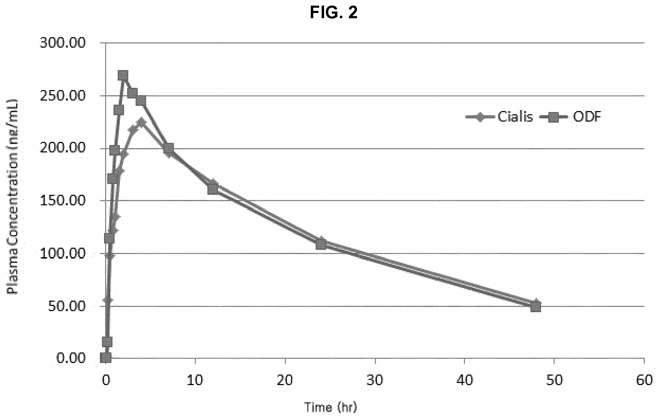

Bioequivalence test was carried out using the orally disintegrating film formulation prepared in Example 1-1 and the comparative formulation (i.e., Cialis 20mg Tablet). Specifically, the orally disintegrating film formulation of Example 1-1 was administered to the oral cavity of fasted volunteers (n=8). The volunteers completely dissolved the formulation in the oral cavity and then swallowed. Blood samples were taken at 0 minute, 5 minutes, 15 minutes, 30 minutes, 45 minutes, 1 hour, 1.5 hour, 2 hours, 3 hours, 4 hours, 7 hours, 12 hours, 24 hours, and 48 hours and then the concentrations thereof were analyzed with a LC-MS-MS method. The plasma concentration profiles are shown in FIG. 2 (ODF: the Example 1-1 formulation, Cialis: the comparative formulation). The ratios of pharmacokinetic parameters calculated therefrom (the Example 1-1 formulation/the comparative formulation) are shown in the following Table 4.

**<Table 4>**

| | Parameter | Value | 90% CI |
|---|---|---|---|
| The Example 1-1 formulation / The comparative formulation | AUC | 0.943 | 0.8645∼1.0294 |
| | Cmax | 1.011 | 0.8451∼1.2099 |

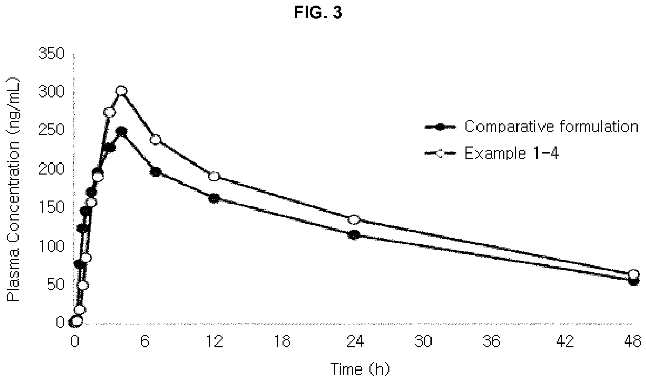

And also, bioequivalence test was carried out using the Example 1-4 formulation which was prepared by using only pullulan as a film forming agent and the comparative formulation (i.e., Cialis 20mg Tablet), according to the same methods. The plasma concentration profiles are shown in FIG. 3. The ratios of pharmacokinetic parameters calculated therefrom (the Example 1-4 formulation/the comparative formulation) are shown in the following Table 5.

**<Table 5>**

| | Parameter | Value | 90% CI |
|---|---|---|---|
| The Example 1-4 formulation / The comparative formulation | AUC | 1.1511 | 1.0096∼1.3125 |
| | Cmax | 1.2554 | 1.0067∼1.5656 |

As shown in the Tables 4 and 5 & FIGs. 2 and 3, the orally disintegrating film formulation of the present invention exhibited bioequivalence to the Cialis 20mg Tablet. However, the Example 1-4 formulation which was prepared by using only pullulan as a film forming agent did not exhibited bioequivalence to the Cialis 20mg Tablet.

## Claims

1. An orally disintegrating film formulation, comprising tadalafil as an active ingredient and a combination of pullulan and polyvinylpyrrolidone as a film forming agent.

2. The orally disintegrating film formulation according to claim 1, wherein said tadalafil has d₉₀ particle size of 40 µm or less, optionally 5 to 25 µm.

3. The orally disintegrating film formulation according to claim 1, wherein said pullulan has weight average molecular weight ranging from 50,000 to 200,000.

4. The orally disintegrating film formulation according to claim 1, wherein said polyvinylpyrrolidone has weight average molecular weight ranging from 700,000 to 2,500,000.

5. The orally disintegrating film formulation according to claim 1, wherein the weight ratio of tadalafil, pullulan and polyvinylpyrrolidone is 1 : 1.8∼2.5 : 0.8∼1.3, optionally 1 : 2 : 1.

6. The orally disintegrating film formulation according to claim 1, further comprising hydroxypropyl cellulose as a solubilizing agent.

7. The orally disintegrating film formulation according to claim 6, wherein said hydroxypropyl cellulose is present in 1.5∼3 %(w/w) based on the total weight of the orally disintegrating film formulation.

8. The orally disintegrating film formulation according to any one of claims 1 to 7, further comprising one or more pharmaceutically acceptable excipients selected from the group consisting of a surfactant, a plasticizer, a sweetening agent, a flavoring agent, and a coloring agent.

9. The orally disintegrating film formulation according to claim 8, wherein the surfactant is sodium lauryl sulfate; the plasticizer is polyethylene glycol; and the sweetening agent is a mixture of sucralose and mannitol.

10. The orally disintegrating film formulation according to claim 9, comprising tadalafil 15∼20 %(w/w), pullulan 30∼40 %(w/w), polyvinylpyrrolidone 15∼20 %(w/w), hydroxypropyl cellulose 1.5∼3 %(w/w), a surfactant 0.3∼1.0 %(w/w), a plasticizer 3∼7 %(w/w), a sweetening agent 18∼23 %(w/w), a flavoring agent 1.5∼3 %(w/w), and a coloring agent 1.5∼3 %(w/w), per unit formulation.

11. The orally disintegrating film formulation according to claim 9, comprising tadalafil 20 mg, pullulan 40 mg, polyvinylpyrrolidone 20 mg, hydroxypropyl cellulose 2 mg, a surfactant 0.5 mg, a plasticizer 5 mg, a sweetening agent 22 mg, a flavoring agent 2 mg, and a coloring agent 2 mg, per unit formulation.

12. A process for preparing an orally disintegrating film formulation, which comprises:
(a) dissolving or dispersing tadalafil, pullulan, polyvinylpyrrolidone, optionally a solubilizing agent, and a pharmaceutically acceptable excipient in ethanol or a mixture of ethanol and water to form a solution or a dispersion; and
(b) casting the solution or the dispersion obtained in the step (a) on a hydrophobic film, followed by drying the resultant.

13. The process according to claim 12, wherein the solubilizing agent is hydroxypropyl cellulose.

14. The process according to claim 12, wherein the weight ratio of ethanol and water in the mixture of ethanol and water is 1 : 2.5∼5.0.

15. The process according to claim 12, wherein the step (b) is performed by degassing the solution or the dispersion obtained in the step (a) to remove an air bubble, casting the resulting solution or dispersion on a hydrophobic film, and then drying the resultant.

## Patentansprüche

1. Schmelzfilmformel, umfassend Tadalafil als Wirkstoff und eine Kombination aus Pullulan und Polyvinylpyrrolidon als filmbildendes Mittel.

2. Schmelzfilmformel nach Anspruch 1, worin dieses Tadalafil eine d₉₀-Partikelgröße von 40 µm oder weniger - optional 5 bis 25 µm - aufweist.

3. Schmelzfilmformel nach Anspruch 1, worin dieses Pullulan ein gewichtsgemitteltes Molekulargewicht von 50.000 bis 200.000 aufweist.

4. Die Schmelzfilmformel nach Anspruch 1, worin dieses Polyvinylpyrrolidon ein gewichtsgemitteltes Molekulargewicht von 700.000 bis 2.500.000 aufweist.

5. Schmelzfilmformel nach Anspruch 1, worin das Gewichtsverhältnis von Tadalafil, Pullulan und Polyvinylpyrrolidon 1: 1,8-2,5: 0,8-1,3 ist optional 1: 2: 1.

6. Schmelzfilmformel nach Anspruch 1, ferner umfassend Hydroxypropylcellulose als Lösungshilfsmittel.

7. Schmelzfilmformel nach Anspruch 6, worin diese Hydroxypropylcellulose in einer Menge von 1,5-3 % (Gew./Gew.) basierend auf dem Gesamtgewicht der Schmelzfilmformel vorliegt.

8. Schmelzfilmformel nach einem der vorherigen Ansprüche 1 bis 7, ferner ein oder mehrere pharmazeutisch akzeptable Trägerstoffe umfassend, die aus einer Gruppe, bestehend aus einem Tensid, einem Weichmacher, einem Süßungsmittel, einem Geschmacksstoff und einem Farbstoff ausgewählt ist.

9. Schmelzfilmformel nach Anspruch 8, worin das Tensid Natriumlaurylsulfat, der Weichmacher Polyethyleneglycol und das Süßungsmittel eine Mischung aus Sucralose und Mannitol ist.

10. Schmelzfilmformel nach Anspruch 9, umfassend 15-20 % (Gew./Gew.) Tadalafil, 30-40 % (Gew./Gew.) Pullulan, 15-20 % (Gew./Gew.) Polyvinylpyrrolidon, 1,5-3 % (Gew./Gew.) Hydroxypropylcellulose, 0,3-1,0 % (Gew./Gew.) Tenside, 3-7 % (Gew./Gew.) Weichmacher, 18-23 % (Gew./Gew.) Süßungsmittel, 1,5-3 % (Gew./Gew.) Geschmacksstoffe und 1,5-3 % (Gew./Gew.) Farbstoffe pro Formeleinheit.

11. Schmelzfilmformel nach Anspruch 9, umfassend 20 mg Tadalafil, 40 mg Pullulan, 20 mg Polyvinylpyrrolidon, 2 mg Hydroxypropylcellulose, 0,5 mg Tenside, 5 mg Weichmacher, 22 mg Süßungsmittel, 2 mg Geschmacksstoffe und 2 mg Farbstoffe pro Formulierungseinheit.

12. Prozess zur Vorbereitung einer Schmelzfilmformel, umfassend:
(a) Auflösen oder Dispergieren von Tadalafil, Pullulan, Polyvinylpyrrolidon, eines optionalen Lösungshilfsmittels und eines pharmazeutisch akzeptablen Trägerstoffs in Ethanol und Wasser, um eine Lösung oder Dispersion zu bilden; und
(b) Gießen der Lösung oder Dispersion, die in Schritt (a) gewonnen wurde, auf einen hydrophoben Film und anschließendes Trocknen des resultierenden Produkts.

13. Prozess nach Anspruch 12, worin das Lösungshilfsmittel Hydroxypropylcellulose ist.

14. Prozess nach Anspruch 12, worin das Gewichtsverhältnis von Ethanol und Wasser 1: 2,5-5,0 ist.

15. Prozess nach Anspruch 12, worin der Schritt (b) das Entgasen der Lösung oder Dispersion, die in Schritt (a) gewonnen wurde, zum Entfernen von Luftblasen, das Gießen der resultierenden Lösung oder Dispersion auf einen hydrophoben Film und das anschließende Trocknen des resultierenden Produkts umfasst.

## Revendications

1. Formulation de film à désintégration orale, comprenant du tadalafil en tant que principe actif et une combinaison de pullulane et de polyvinylpyrrolidone en tant qu'agent filmogène.

2. Formulation de film à désintégration orale selon la revendication 1, dans laquelle ledit tadalafil a une taille de particule d₉₀ de 40 µm ou moins, facultativement de 5 à 25 µm.

3. Formulation de film à désintégration orale selon la revendication 1, dans laquelle ledit pullulane a un poids moléculaire moyen en poids compris entre 50 000 et 200 000.

4. Formulation de film à désintégration orale selon la revendication 1, dans laquelle ladite polyvinylpyrrolidone a un poids moléculaire moyen en poids allant de 700 000 à 2 500 000.

5. Formulation de film à désintégration orale selon la revendication 1, dans laquelle le rapport en poids du tadalafil, du pullulane et de la polyvinylpyrrolidone est de 1 : 1,8 à 2,5 : 0,8 à 1,3 facultativement 1 : 2 : 1.

6. Formulation de film à désintégration orale selon la revendication 1, comprenant en outre de l'hydroxypropylcellulose en tant qu'agent de solubilisation.

7. Formulation de film à désintégration orale selon la revendication 6, dans laquelle ladite hydroxypropylcellulose est présente dans 1,5 à 3 % (p/p) sur la base du poids total de la formulation de film à désintégration orale.

8. Formulation de film à désintégration orale selon l'une quelconque des revendications 1 à 7, comprenant en outre un ou plusieurs excipients pharmaceutiquement acceptables choisis dans le groupe constitué d'un tensioactif, d'un plastifiant, d'un édulcorant, d'un agent aromatisant et d'un agent colorant.

9. Formulation de film à désintégration orale selon la revendication 8, dans laquelle le tensioactif est du laurylsulfate de sodium ; le plastifiant est du polyéthylène glycol ; et l'agent édulcorant est un mélange de sucralose et de mannitol.

10. Formulation de film à désintégration orale selon la revendication 9, comprenant 15 à 20 % de tadalafil (p/p), 30 à 40 % de pullulane (p/p), 15 à 20 % de polyvinylpyrrolidone (p/p), 1,5 à 3 % d'hydroxypropylcellulose (p/p), 0,3 à 1,0 % d'agent tensio-actif (p/p), 3 à 7 % de plastifiant (p/p), 18 à 23 % d'édulcorant (p/p), 1,5 à 3 % d'agent aromatisant (p/p), et 1,5 à 3 % d'agent colorant (p/p), par formulation unitaire.

11. Formulation de film à désintégration orale selon la revendication 9, comprenant 20 mg de tadalafil, 40 mg de pullulane, 20 mg de polyvinylpyrrolidone, 2 mg d'hydroxypropylcellulose, 0,5 mg d'un agent tensioactif, 5 mg d'un agent plastifiant, 22 mg d'un agent édulcorant, 2 mg d'un agent aromatisant, et 2 mg d'un colorant, par formulation unitaire.

12. Procédé de préparation d'une formulation de film à désintégration orale, qui comprend :
(a) la dissolution ou la dispersion du tadalafil, du pullulane, de la polyvinylpyrrolidone, éventuellement un agent solubilisant, et un excipient pharmaceutiquement acceptable dans l'éthanol ou un mélange d'éthanol et d'eau pour former une solution ou une dispersion ; et
(b) la coulée de la solution ou de la dispersion obtenue à l'étape (a) sur un film hydrophobe, suivi du séchage de la résultante.

13. Procédé selon la revendication 12, dans lequel l'agent de solubilisation est l'hydroxypropylcellulose.

14. Procédé selon la revendication 12, dans lequel le rapport pondéral de l'éthanol et de l'eau dans le mélange d'éthanol et d'eau est de 1 : 2,5 à 5,0.

15. Procédé selon la revendication 12, dans lequel l'étape (b) est réalisée par le dégazage de la solution ou de la dispersion obtenue à l'étape (a) pour éliminer une bulle d'air, la coulée de la solution ou de la dispersion résultante sur un film hydrophobe, puis le séchage de la résultante.
